Europäisches Patentamt
European Patent Office
Office européen des brevets

(19)

(11) Publication number: **0 571 856 A1**

(12) # EUROPEAN PATENT APPLICATION

(21) Application number: **93107997.4**

(51) Int. Cl.[5]: **C07D 239/60**, A01N 43/54

(22) Date of filing: **17.05.93**

(30) Priority: **29.05.92 JP 161707/92**
**25.09.92 JP 279180/92**

(43) Date of publication of application:
**01.12.93 Bulletin 93/48**

(84) Designated Contracting States:
**BE CH DE FR GB IT LI NL**

(71) Applicant: **NIHON BAYER AGROCHEM K.K.**
**10-8, Takanawa 4-chome**
**Minato-ku**
**Tokyo 108(JP)**

(72) Inventor: **Goto, Toshio**
**214-18, Koganei,**
**Kokubunji-machi**
**Shimotsuga-gun, Tochigi(JP)**
Inventor: **Kitagawa, Yoshinori**
**1085, Ara-machi**
**Moka-shi, Tochigi(JP)**
Inventor: **Hayakawa, Hidenori**
**3-17-20, Ekiminami-machi**
**Oyama-shi, Tochigi(JP)**
Inventor: **Shibuya, Katsuhiko**
**1425-2, Hitotonoya,**
**Oaza**
**Oyama-shi, Tochigi(JP)**
Inventor: **Watanabe, Ryo**
**1057-1, Inabago,**
**Oaza**
**Oyama-shi, Tochigi(JP)**

(74) Representative: **Schumacher, Günter, Dr. et al**
**Bayer AG**
**Konzernverwaltung RP**
**Patentabteilung**
**D-51368 Leverkusen Bayerwerk (DE)**

(54) **Pyrimidinylthioacrylic acid derivatives useful as herbicides.**

(57) The present invention relates to novel pyrimidinylthioacrylic acid derivatives of the formula (I)

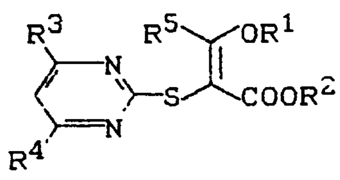

(I)

wherein

$R^1$ represents hydrogen or in each case optionally substituted $C_{1-6}$ alkyl, $C_{2-4}$ alkenyl or $C_{2-4}$ alkynyl,

$R^2$ represents hydrogen or optionally substituted $C_{1-6}$ alkyl, or alkali salts,

$R^3$ and $R^4$ each independently represent $C_{1-4}$ alkyl, $C_{1-4}$ alkoxy, halogeno-$C_{1-4}$ alkyl, halogeno-$C_{1-4}$ alkoxy or halogen, and

$R^5$ represents hydrogen, or in each case optionally substituted $C_{1-6}$ alkyl, phenyl, $C_{2-4}$ alkenyl, $C_{2-4}$ alkynyl, or a heterocyclic ring, or an alkali metal salt, alkali earth metal or organic ammonium salt of pyrimidinylthioacrylic acid derivatives of the formula (I),

to processes for their preparation and their use as herbicides, as well as to novel intermediates and to a process for their preparation.

EP 0 571 856 A1

The present invention relates to novel pyrimidinylthioacrylic acid derivatives, to processes for their preparation, and to their use as herbicides, as well as to novel intermediates and to a process for their preparation.

It has already been disclosed that a certain group of pyrimidinylthiocarboxylic acid derivatives is useful as herbicides (see Japanese Laid-Open Patent Application Nos. 52872/1991 and 135964/1991).

There have now been found novel pyrimidinylthioacrylic acid derivatives of the formula (I)

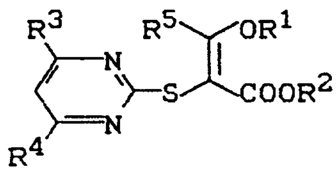

(I)

wherein

- $R^1$ represents hydrogen or in each case optionally substituted $C_{1-6}$ alkyl, $C_{2-4}$ alkenyl or $C_{2-4}$ alkynyl,
- $R^2$ represents hydrogen or optionally substituted $c_{1-6}$ alkyl or alkali salts,
- $R^3$ and $R^4$ each independently represent $C_{1-4}$ alkyl, $C_{1-4}$ alkoxy, halogeno-$C_{1-4}$ alkyl, halogeno-$C_{1-4}$ alkoxy or halogen, and
- $R^5$ represents hydrogen, or in each case optionally substituted $C_{1-6}$ alkyl, phenyl, $C_{2-4}$ alkenyl, $C_{2-4}$ alkynyl, or a heterocyclic ring, or represents an alkali metal salt, alkali earth metal or organic ammonium salt of pyrimidinylthioacrylic acid derivatives of the formula (I).

Pyrimidinylthioacrylic acid derivatives of the formula (I) are obtained when

(a) in the case where $R^1$ and $R^5$ represent hydrogen; compounds of the formula (II)

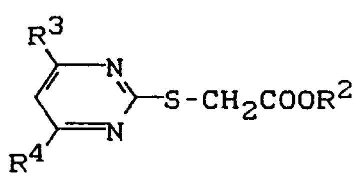

(II)

wherein $R^2$, $R^3$ and $R^4$ represent the same meanings as mentioned above,
are reacted with a formylating agent, in the presence of inert solvents,

or

(b) in the case where $R^1$ has the meanings mentioned above, except hydrogen;
compounds of the formula (Ia)

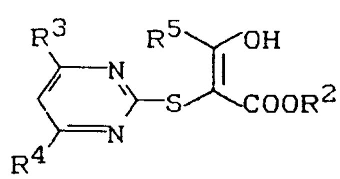

(Ia)

wherein $R^2$, $R^3$, $R^4$ and $R^5$ represent the same meanings as mentioned above,
are reacted with compounds of the formula (III)

$R^6$ - X (III)

wherein $R^6$ has the meanings as $R^1$, except hydrogen, and X represents $-O-SO_2-M$, in which M represents alkyl or aryl,
in the presence of inert solvents,

or

(c) in the case where $R^1$ represents hydrogen, and $R^5$ has the meanings mentioned above, except hydrogen; compounds of the formula (IV)

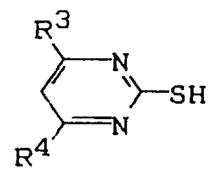

(IV)

wherein $R^2$ and $R^3$ have the same meanings as mentioned above,
are reacted with compounds of the formula (V)

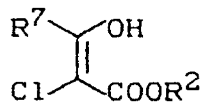

(V)

wherein $R^2$ has the same meaning as mentioned above and $R^7$ has the same meaning as $R^5$ except hydrogen,
in the presence of inert solvents and if appropriate, in the presence of acid binder,
(d) in the case where $R^2$ represents hydrogen; compounds of the formula (Ib)

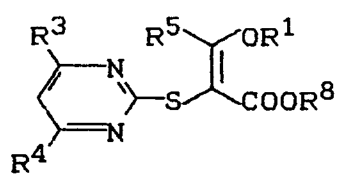

(Ib)

wherein $R^1$, $R^3$, $R^4$ and $R^5$ have the same meanings as mentioned above, and $R^8$ represents optionally substituted $C_{1-6}$ alkyl,
are hydrolyzed,
in the presence of inert solvents and, if appropriate, in the presence of acid binder.

The novel pyrimidinylthioacrylic acid derivatives according to the invention exhibit powerful herbicidal properties.

Surprisingly, the pyrimidinylthioacrylic acid derivatives according to the invention exhibit a substantially higher selective herbicidal activity than those known from the prior art, for instance, the aforementioned Japanese Laid-Open Patent Application Nos. 52872/1991 and 135964/1991.

In the compounds of the formula (I) and their intermediates, the $C_{1-6}$ alkyl group represents straight chain or branched alkyl such as methyl, ethyl, n-propyl, isopropyl, or n-(iso-, sec- or tert-)butyl, n-pentyl, n-hexyl, preferably methyl, ethyl, n-propyl, isopropyl or n-(iso-, sec- or tert-)butyl.

The $C_{1-4}$ alkyl moiety represents straight chain or branched alkyl as illustrated in the paragraph directed to the explanation of the $C_{1-6}$ alkyl.

The $C_{2-4}$ alkenyl includes vinyl, allyl, 1-propenyl, 1-(2-, or 3-)butenyl, and the like, preferably vinyl, allyl and 1-propenyl.

The $C_{2-4}$ alkynyl includes ethynyl, propargyl, 1-propenyl and the like, preferably propargyl and 1-propynyl.

The halogen or halogen moiety represents fluorine, chlorine, bromine or iodine, preferably fluorine, chlorine or bromine.

The 5-membered heterocyclic ring having one or two hetero atoms selected from the group consisting of nitrogen, sulfur and oxygen, such as thienyl, furyl, thiazolyl, isothiazolyl, pyrrolyl, imidazolyl, pyrazolyl

and the like.

The 6-membered heterocyclic ring having one or two hetero atoms selected from the group consisting of nitrogen, sulfur and oxygen, such as pyridyl, pyrazinyl, pyrimidinyl, pyridazinyl and the like.

Among the pyrimidinylthioacrylic acid derivatives according to the invention of the formula (I), the preferred compounds are those in which

| | |
|---|---|
| $R^1$ | represents hydrogen, $C_{1-6}$ alkyl which are unsubstituted or substituted by at least one substituent selected from the group consisting of halogen, $C_{1-4}$ alkoxy, $C_{1-4}$ alkylthio, acyl, optionally substituted phenyl or 5- or 6-membered heterocyclic rings having one or two hetero atoms selected from the group consisting of nitrogen, sulfur and oxygen, |
| $R^1$ | further represents $C_{2-4}$ alkenyl, $C_{2-4}$ halogenoalkenyl, $C_{2-4}$ alkynyl and $C_{2-4}$ halogenoalkynyl, |
| $R^2$ | represents hydrogen, $C_{1-4}$ alkyl, $C_{1-2}$ alkoxy-$C_{1-2}$ alkyl, benzyl or alkali salts, |
| $R^3$ and $R^4$ | each independently represent methyl, ethyl, methoxy, ethoxy, halogeno-$C_{1-2}$ alkyl, halogeno-$C_{1-2}$ alkoxy or chlorine, and |
| $R^5$ | represents hydrogen, optionally substituted $C_{1-6}$ alkyl, suitable substituents are selected from the group consisting of halogen, $C_{1-4}$ alkoxy, $C_{1-4}$ alkylthio, acyl and phenyl which may be substituted, $R^5$ further represents phenyl which is unsubstituted or substituted at least by one substituent selected from the group consisting of halogen, $C_{1-4}$ alkyl and $C_{1-4}$ alkoxy, $R^5$ further represents $C_3$ alkenyl, $C_3$ alkynyl or a 5- or 6-membered heterocyclic ring having one or two hetero atoms selected from the group consisting of nitrogen, sulfur and oxygen and which may be substituted. |

Very particularly preferred pyrimidinylthioacrylic acid derivatives according to the invention of the formula (I) are those in which

| | |
|---|---|
| $R^1$ | represents hydrogen, optionally substituted $C_{1-4}$ alkyl, suitable substituents are selected from the group consisting of fluorine, chlorine, methoxy, ethoxy, methylthio, ethylthio, acetyl, pyridyl, thiazolyl or phenyl which are unsubstituted or substituted by at least one substituent selected from the group consisting of halogen, $C_{1-4}$ alkyl and $C_{1-2}$ alkoxy, |
| $R^1$ | further represents $C_{2-3}$ alkenyl, $C_{2-3}$ chloroalkenyl, $C_3$ alkynyl, and $C_3$-chloroalkynyl, |
| $R^2$ | represents hydrogen, methyl, ethyl, benzyl or alkali salts, |
| $R^3$ and $R^4$ | represent methoxy, and |
| $R^5$ | represents hydrogen, optionally substituted $C_{1-4}$ alkyl, suitable substituents are selected from the group consisting of fluorine, chlorine, methoxy, ethoxy, methylthio and ethylthio or $R^5$ further represents phenyl which is unsubstituted or substituted by at least one substituent selected from the group consisting of fluorine, chlorine, methyl and methoxy or $R^5$ further represents allyl, propargyl, pyridyl, thienyl, or furyl. |

As examples of the compounds of the formula (I) according to the invention, there may be mentioned in the following table 1, in addition to the compounds that will be enumerated in the Examples hereinafter.

Table 1

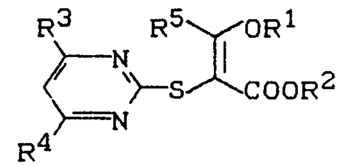

| $R^1$ | $R^2$ | $R^3$ | $R^4$ | $R^5$ |
|---|---|---|---|---|
| hydrogen | methyl | methoxy | methoxy | hydrogen |
| hydrogen | ethyl | methoxy | methoxy | hydrogen |
| hydrogen | n-propyl | methoxy | methoxy | hydrogen |
| hydrogen | n-butyl | methoxy | methoxy | hydrogen |
| hydrogen | n-pentyl | methoxy | methoxy | hydrogen |
| hydrogen | hydrogen | methoxy | methoxy | hydrogen |
| methyl | hydrogen | methoxy | methoxy | hydrogen |
| methyl | methyl | methoxy | methoxy | hydrogen |
| methyl | ethyl | methoxy | methoxy | hydrogen |
| methyl | n-propyl | methoxy | methoxy | hydrogen |
| methyl | n-butyl | methoxy | methoxy | hydrogen |
| ethyl | hydrogen | methoxy | methoxy | hydrogen |
| ethyl | methyl | methoxy | methoxy | hydrogen |

Table 1 (continued)

| $R^1$ | $R^2$ | $R^3$ | $R^4$ | $R^5$ |
|---|---|---|---|---|
| ethyl | ethyl | methoxy | methoxy | hydrogen |
| n-propyl | hydrogen | methoxy | methoxy | hydrogen |
| n-propyl | methyl | methoxy | methoxy | hydrogen |
| n-propyl | ethyl | methoxy | methoxy | hydrogen |
| isopropyl | hydrogen | methoxy | methoxy | hydrogen |
| isopropyl | methyl | methoxy | methoxy | hydrogen |
| isopropyl | ethyl | methoxy | methoxy | hydrogen |
| isopropyl | n-propyl | methoxy | methoxy | hydrogen |
| n-butyl | hydrogen | methoxy | methoxy | hydrogen |
| n-butyl | methyl | methoxy | methoxy | hydrogen |
| n-butyl | ethyl | methoxy | methoxy | hydrogen |
| iso-butyl | hydrogen | methoxy | methoxy | hydrogen |
| iso-butyl | methyl | methoxy | methoxy | hydrogen |
| iso-butyl | ethyl | methoxy | methoxy | hydrogen |
| sec-butyl | hydrogen | methoxy | methoxy | hydrogen |
| sec-butyl | methyl | methoxy | methoxy | hydrogen |
| sec-butyl | ethyl | methoxy | methoxy | hydrogen |

Table 1 (continued)

| $R^1$ | $R^2$ | $R^3$ | $R^4$ | $R^5$ |
|---|---|---|---|---|
| n-pentyl | hydrogen | methoxy | methoxy | hydrogen |
| n-pentyl | methyl | methoxy | methoxy | hydrogen |
| n-pentyl | ethyl | methoxy | methoxy | hydrogen |
| iso-pentyl | hydrogen | methoxy | methoxy | hydrogen |
| iso-pentyl | methyl | methoxy | methoxy | hydrogen |
| iso-pentyl | ethyl | methoxy | methoxy | hydrogen |
| sec-pentyl | hydrogen | methoxy | methoxy | hydrogen |
| sec-pentyl | methyl | methoxy | methoxy | hydrogen |
| sec-pentyl | ethyl | methoxy | methoxy | hydrogen |
| acetonyloxy | hydrogen | methoxy | methoxy | hydrogen |
| acetonyloxy | methyl | methoxy | methoxy | hydrogen |
| 2-chloro-ethyl | hydrogen | methoxy | methoxy | hydrogen |
| 2-chloro-ethyl | methyl | methoxy | methoxy | hydrogen |
| benzyl | hydrogen | methoxy | methoxy | hydrogen |
| benzyl | methyl | methoxy | methoxy | hydrogen |
| benzyl | ethyl | methoxy | methoxy | hydrogen |
| 4-chloro-benzyl | hydrogen | methoxy | methoxy | hydrogen |

Table 1 (continued)

| $R^1$ | $R^2$ | $R^3$ | $R^4$ | $R^5$ |
|---|---|---|---|---|
| 4-chloro-benzyl | methyl | methoxy | methoxy | hydrogen |
| 2-chloro-benzyl | hydrogen | methoxy | methoxy | hydrogen |
| 2-chloro-benzyl | methyl | methoxy | methoxy | hydrogen |
| 2,4-dichloro-benzyl | hydrogen | methoxy | methoxy | hydrogen |
| 2,4-dichloro-benzyl | methyl | methoxy | methoxy | hydrogen |
| 4-methyl-benzyl | hydrogen | methoxy | methoxy | hydrogen |
| 4-methyl-benzyl | methyl | methoxy | methoxy | hydrogen |
| allyl | hydrogen | methoxy | methoxy | hydrogen |
| allyl | methyl | methoxy | methoxy | hydrogen |
| propargyl | hydrogen | methoxy | methoxy | hydrogen |
| propargyl | methyl | methoxy | methoxy | hydrogen |
| hydrogen | methyl | methoxy | methoxy | methyl |
| hydrogen | methyl | methoxy | methoxy | ethyl |
| hydrogen | methyl | methoxy | methoxy | n-propyl |
| hydrogen | methyl | methoxy | methoxy | isopropyl |
| hydrogen | methyl | methoxy | methoxy | n-butyl |
| hydrogen | methyl | methoxy | methoxy | iso-butyl |

Table 1 (continued)

| $R^1$ | $R^2$ | $R^3$ | $R^4$ | $R^5$ |
|---|---|---|---|---|
| hydrogen | methyl | methoxy | methoxy | sec-butyl |
| hydrogen | methyl | methoxy | methoxy | t-butyl |
| hydrogen | methyl | methoxy | methoxy | n-pentyl |
| hydrogen | methyl | methoxy | methoxy | 2-bromoethyl |
| hydrogen | methyl | methoxy | methoxy | 3,3,3-tri-fluoroethyl |
| hydrogen | methyl | methoxy | methoxy | allyl |
| hydrogen | methyl | methoxy | methoxy | propargyl |
| hydrogen | ethyl | methoxy | methoxy | methyl |
| hydrogen | ethyl | methoxy | methoxy | ethyl |
| hydrogen | ethyl | methoxy | methoxy | n-propyl |
| hydrogen | ethyl | methoxy | methoxy | isopropyl |
| hydrogen | ethyl | methoxy | methoxy | n-butyl |
| hydrogen | ethyl | methoxy | methoxy | iso-butyl |
| hydrogen | n-propyl | methoxy | methoxy | sec-butyl |
| hydrogen | ethyl | methoxy | methoxy | t-butyl |
| hydrogen | benzyl | methoxy | methoxy | t-butyl |
| hydrogen | t-butyl | methoxy | methoxy | t-butyl |

Table 1 (continued)

| $R^1$ | $R^2$ | $R^3$ | $R^4$ | $R^5$ |
|---|---|---|---|---|
| hydrogen | ethyl | methoxy | methoxy | t-butyl |
| hydrogen | n-propyl | methoxy | methoxy | t-butyl |
| hydrogen | isopropyl | methoxy | methoxy | t-butyl |
| hydrogen | hydrogen | methoxy | methoxy | t-butyl |
| hydrogen | methyl | methoxy | methoxy | methoxymethyl |
| hydrogen | ethyl | methoxy | methoxy | methoxymethyl |
| methyl | methyl | methoxy | methoxy | methoxymethyl |
| methyl | ethyl | methoxy | methoxy | methoxymethyl |
| hydrogen | hydrogen | methoxy | methoxy | phenyl |
| hydrogen | methyl | methoxy | methoxy | phenyl |
| hydrogen | ethyl | methoxy | methoxy | phenyl |
| hydrogen | methyl | methoxy | methoxy | 2-thienyl |
| hydrogen | ethyl | methoxy | methoxy | 2-thienyl |
| hydrogen | methyl | methoxy | methoxy | 3-thienyl |
| hydrogen | ethyl | methoxy | methoxy | 3-thienyl |
| hydrogen | methyl | methoxy | methoxy | 2-furyl |
| hydrogen | methyl | methoxy | methoxy | 4-chloro-phenyl |

Table 1 (continued)

| $R^1$ | $R^2$ | $R^3$ | $R^4$ | $R^5$ |
|---|---|---|---|---|
| hydrogen | methyl | methoxy | methoxy | 4-chloro-phenyl |
| hydrogen | methyl | methoxy | methoxy | 4-chloro-phenyl |
| hydrogen | hydrogen | methoxy | methoxy | 3,5-dichloro-phenyl |
| methyl | hydrogen | methoxy | methoxy | methyl |
| methyl | methyl | methoxy | methoxy | methyl |
| methyl | ethyl | methoxy | methoxy | methyl |
| ethyl | methyl | methoxy | methoxy | methyl |
| ethyl | ethyl | methoxy | methoxy | methyl |
| n-propyl | methyl | methoxy | methoxy | methyl |
| n-propyl | ethyl | methoxy | methoxy | methyl |
| isopropyl | methyl | methoxy | methoxy | methyl |
| isopropyl | ethyl | methoxy | methoxy | methyl |
| 2-bromoethyl | methyl | methoxy | methoxy | methyl |
| 2-bromoethyl | ethyl | methoxy | methoxy | methyl |
| allyl | methyl | methoxy | methoxy | methyl |
| allyl | ethyl | methoxy | methoxy | methyl |
| propargyl | hydrogen | methoxy | methoxy | methyl |

Table 1 (continued)

| $R^1$ | $R^2$ | $R^3$ | $R^4$ | $R^5$ |
|---|---|---|---|---|
| propargyl | methyl | methoxy | methoxy | methyl |
| propargyl | ethyl | methoxy | methoxy | methyl |
| n-butyl | methyl | methoxy | methoxy | methyl |
| sec-butyl | methyl | methoxy | methoxy | methyl |
| benzyl | hydrogen | methoxy | methoxy | methyl |
| benzyl | methyl | methoxy | methoxy | methyl |
| benzyl | ethyl | methoxy | methoxy | methyl |
| 4-chlorobenzyl | ethyl | methoxy | methoxy | methyl |
| 3-chlorobenzyl | ethyl | methoxy | methoxy | methyl |
| 4-methylbenzyl | ethyl | methoxy | methoxy | methyl |
| 3,5-dimethylbenzyl | ethyl | methoxy | methoxy | methyl |
| 4-methoxybenzyl | ethyl | methoxy | methoxy | methyl |
| 2-chloro-5-pyridylmethyl | hydrogen | methoxy | methoxy | methyl |
| 2-chloro-5-pyridylmethyl | methyl | methoxy | methoxy | methyl |
| 2-chloro-5-pyridylmethyl | ethyl | methoxy | methoxy | methyl |
| 5-chloro-2-thiazolylmethyl | methyl | methoxy | methoxy | methyl |

Table 1 (continued)

| $R^1$ | $R^2$ | $R^3$ | $R^4$ | $R^5$ |
|---|---|---|---|---|
| 5-chloro-2-thiazolylmethyl | ethyl | methoxy | methoxy | methyl |
| 3-pyridylmethyl | methyl | methoxy | methoxy | methyl |
| 4-pyridylmethyl | ethyl | methoxy | methoxy | methyl |
| methyl | methyl | methoxy | methoxy | ethyl |
| methyl | ethyl | methoxy | methoxy | ethyl |
| methyl | methyl | methoxy | methoxy | n-propyl |
| methyl | ethyl | methoxy | methoxy | n-propyl |
| methyl | benzyl | methoxy | methoxy | n-propyl |
| methyl | hydrogen | methoxy | methoxy | n-propyl |
| methyl | methyl | methoxy | methoxy | isopropyl |
| ethyl | methyl | methoxy | methoxy | isopropyl |
| n-propyl | methyl | methoxy | methoxy | isopropyl |
| 2-bromoethyl | methyl | methoxy | methoxy | isopropyl |
| allyl | methyl | methoxy | methoxy | isopropyl |
| propargyl | methyl | methoxy | methoxy | isopropyl |
| propargyl | hydrogen | methoxy | methoxy | isopropyl |
| 4-chlorobenzyl | methyl | methoxy | methoxy | isopropyl |

Table 1 (continued)

| $R^1$ | $R^2$ | $R^3$ | $R^4$ | $R^5$ |
|---|---|---|---|---|
| 3,5-dichlorobenzyl | methyl | methoxy | methoxy | isopropyl |
| 4-nitrobenzyl | methyl | methoxy | methoxy | isopropyl |
| benzyl | hydrogen | methoxy | methoxy | isopropyl |
| allyl | ethyl | methoxy | methoxy | isopropyl |
| propargyl | ethyl | methoxy | methoxy | isopropyl |
| 1-methyl-2-propenyl | methyl | methoxy | methoxy | isopropyl |
| 1-methyl-2-propenyl | ethyl | methoxy | methoxy | isopropyl |
| allyl | hydrogen | methoxy | methoxy | isopropyl |
| methyl | hydrogen | methoxy | methoxy | n-butyl |
| methyl | methyl | methoxy | methoxy | n-butyl |
| methyl | ethyl | methoxy | methoxy | n-butyl |
| ethyl | methyl | methoxy | methoxy | n-butyl |
| methyl | methyl | methoxy | methoxy | iso-butyl |
| n-propyl | methyl | methoxy | methoxy | iso-butyl |
| allyl | methyl | methoxy | methoxy | iso-butyl |
| propargyl | methyl | methoxy | methoxy | iso-butyl |
| methyl | hydrogen | methoxy | methoxy | t-butyl |

Table 1 (continued)

| $R^1$ | $R^2$ | $R^3$ | $R^4$ | $R^5$ |
|---|---|---|---|---|
| methyl | methyl | methoxy | methoxy | t-butyl |
| ethyl | methyl | methoxy | methoxy | t-butyl |
| n-propyl | methyl | methoxy | methoxy | t-butyl |
| 4-chlorobenzyl | methyl | methoxy | methoxy | t-butyl |
| allyl | methyl | methoxy | methoxy | t-butyl |
| propargyl | methyl | methoxy | methoxy | t-butyl |
| 2-chlorobenzyl | ethyl | methoxy | methoxy | t-butyl |
| methyl | methyl | methoxy | methoxy | n-pentyl |
| methyl | ethyl | methoxy | methoxy | n-pentyl |
| t-butyl | methyl | methoxy | methoxy | n-pentyl |
| methyl | methyl | methoxy | methoxy | 2-bromobenzyl |
| n-propyl | methyl | methoxy | methoxy | 2-bromobenzyl |
| methyl | methyl | methoxy | methoxy | 2,2,2-tri-fluoro-ethyl |
| 3-chlorobenzyl | methyl | methoxy | methoxy | 2,2,2-tri-fluoro-ethyl |
| 2,4-difluorobenzyl | methyl | methoxy | methoxy | 2,2,2-tri-fluoro-ethyl |
| methyl | methyl | methoxy | methoxy | allyl |
| ethyl | methyl | methoxy | methoxy | allyl |

Table 1 (continued)

| $R^1$ | $R^2$ | $R^3$ | $R^4$ | $R^5$ |
|---|---|---|---|---|
| 2-bromoethyl | methyl | methoxy | methoxy | allyl |
| methyl | methyl | methoxy | methoxy | propargyl |
| ethyl | methyl | methoxy | methoxy | propargyl |
| ethyl | hydrogen | methoxy | methoxy | propargyl |
| methyl | hydrogen | methoxy | methoxy | phenyl |
| methyl | methyl | methoxy | methoxy | phenyl |
| methyl | ethyl | methoxy | methoxy | phenyl |
| ethyl | methyl | methoxy | methoxy | phenyl |
| ethyl | ethyl | methoxy | methoxy | phenyl |
| n-propyl | methyl | methoxy | methoxy | phenyl |
| n-propyl | ethyl | methoxy | methoxy | phenyl |
| allyl | methyl | methoxy | methoxy | phenyl |
| allyl | ethyl | methoxy | methoxy | phenyl |
| propargyl | methyl | methoxy | methoxy | phenyl |
| propargyl | ethyl | methoxy | methoxy | phenyl |
| 2-bromoethyl | methyl | methoxy | methoxy | phenyl |
| 2,2,2-trifluoro-methyl | methyl | methoxy | methoxy | phenyl |

Table 1 (continued)

| $R^1$ | $R^2$ | $R^3$ | $R^4$ | $R^5$ |
|---|---|---|---|---|
| allyl | methyl | methoxy | methoxy | 4-chlorophenyl |
| propargyl | methyl | methoxy | methoxy | 4-chlorophenyl |
| propargyl | ethyl | methoxy | methoxy | 4-chlorophenyl |
| methyl | methyl | methoxy | methoxy | 4-chlorophenyl |
| ethyl | methyl | methoxy | methoxy | 3-chlorophenyl |
| methyl | methyl | methoxy | methoxy | 3,5-dichloro-phenyl |
| allyl | methyl | methoxy | methoxy | 4-nitrophenyl |
| methyl | methyl | methoxy | methoxy | 2-thienyl |
| methyl | ethyl | methoxy | methoxy | 2-thienyl |
| allyl | methyl | methoxy | methoxy | 2-thienyl |
| propargyl | methyl | methoxy | methoxy | 2-thienyl |
| methyl | methyl | methoxy | methoxy | 3-thienyl |
| ethyl | methyl | methoxy | methoxy | 3-thienyl |
| 4-chlorobenzyl | ethyl | methoxy | methoxy | 3-thienyl |
| 3-chlorobenzyl | methyl | methoxy | methoxy | 3-thienyl |
| 2-chloro-5-pyridylmethyl | hydrogen | methoxy | methoxy | ethyl |
| 2-chloro-5-pyridylmethyl | methyl | methoxy | methoxy | ethyl |

Table 1 (continued)

| $R^1$ | $R^2$ | $R^3$ | $R^4$ | $R^5$ |
|---|---|---|---|---|
| 2-chloro-5-pyridylmethyl | methyl | methoxy | methoxy | n-propyl |
| 2-chloro-5-pyridylmethyl | ethyl | methoxy | methoxy | n-propyl |
| 2-pyridylmethyl | ethyl | methoxy | methoxy | isopropyl |
| 3-pyridylmethyl | methyl | methoxy | methoxy | isopropyl |
| 4-pyridylmethyl | methyl | methoxy | methoxy | isopropyl |
| 2-chloro-5-pyridylmethyl | methyl | methoxy | methoxy | isopropyl |
| 2-chloro-5-pyridylmethyl | methyl | methoxy | methoxy | n-butyl |
| 2-chloro-5-pyridylmethyl | methyl | methoxy | methoxy | iso-butyl |
| 2-thienylmethyl | methyl | methoxy | methoxy | t-butyl |
| 3-pyridylmethyl | methyl | methoxy | methoxy | t-butyl |
| 2-pyridylmethyl | methyl | methoxy | methoxy | t-butyl |
| 4-pyridylmethyl | methyl | methoxy | methoxy | t-butyl |
| 2,3-dichloro-5-pyridylmethyl | methyl | methoxy | methoxy | t-butyl |

Table 1 (continued)

| $R^1$ | $R^2$ | $R^3$ | $R^4$ | $R^5$ |
|---|---|---|---|---|
| 2-chloro-5-pyridylmethyl | methyl | methoxy | methoxy | phenyl |
| 2-chloro-5-pyridylmethyl | ethyl | methoxy | methoxy | phenyl |
| 2-chloro-5-pyridylmethyl | ethyl | methoxy | methoxy | 4-chlorophenyl |
| 2-chloro-5-pyridylmethyl | methyl | methoxy | methoxy | 2-thienyl |
| 2-chloro-5-pyridylmethyl | ethyl | methoxy | methoxy | 2-thienyl |
| 3-pyridylmethyl | methyl | methoxy | methoxy | 2-thienyl |
| 2-pyridylmethyl | ethyl | methoxy | methoxy | 2-thienyl |
| hydrogen | ethyl | methoxy | methoxy | 2-fluorophenyl |

When in the process (a), if, for example, 2-(4,6-dimethoxy-2-pyrimidinylthio)acetic acid methyl ester and methyl formate are used as starting materials, the course of the reaction can be represented by the following equation:

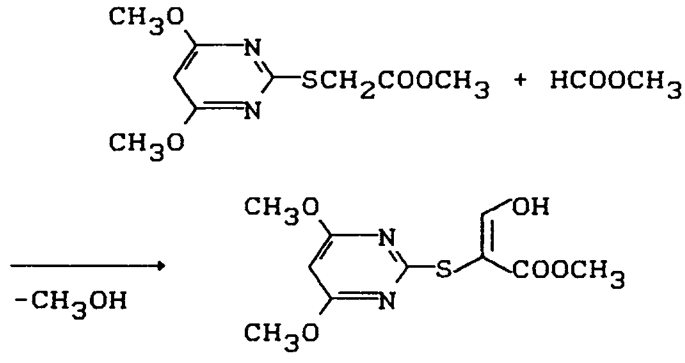


When in the process (b), if, for example, 3-hydroxy-2-(4,6-dimethoxy-2-pyrimidinylthio)acrylic acid methyl ester and methyl sulfate are used as starting materials, the course of the reaction can be represented by the following equation:

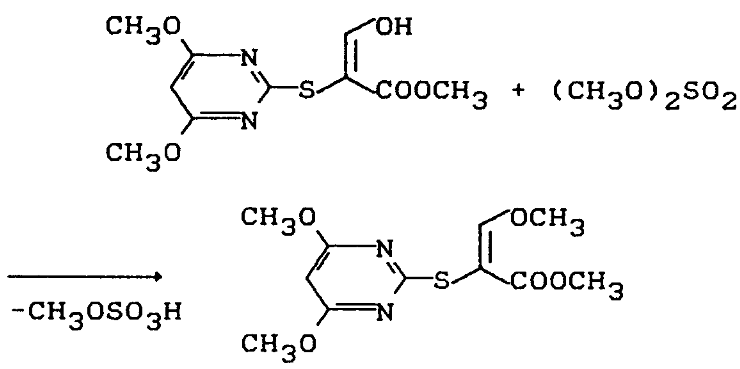


When in the process (c), if, for example, 4,6-dimethoxy-2-mercaptopyrimidine and 2-chloroacetoacetic acid ethyl ester are used as starting materials, the course of the reaction can be represented by the following equation:

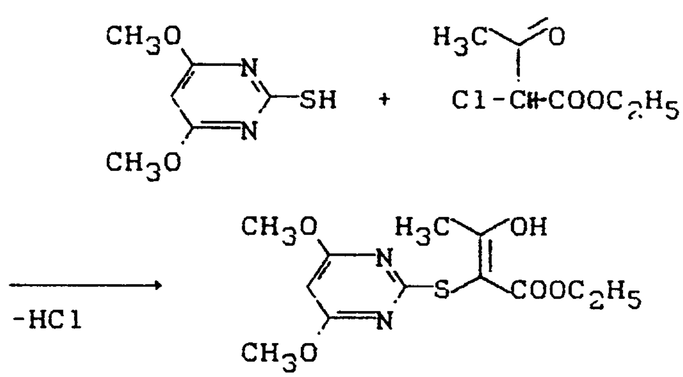


When in the process (d), if, for example, 3-methoxy-2-(4,6-dimethoxy-2-pyrimidinylthio)crotonic acid ethyl ester and sodium hydroxide are used as starting materials, the course of the reaction can be represented by the following equation:

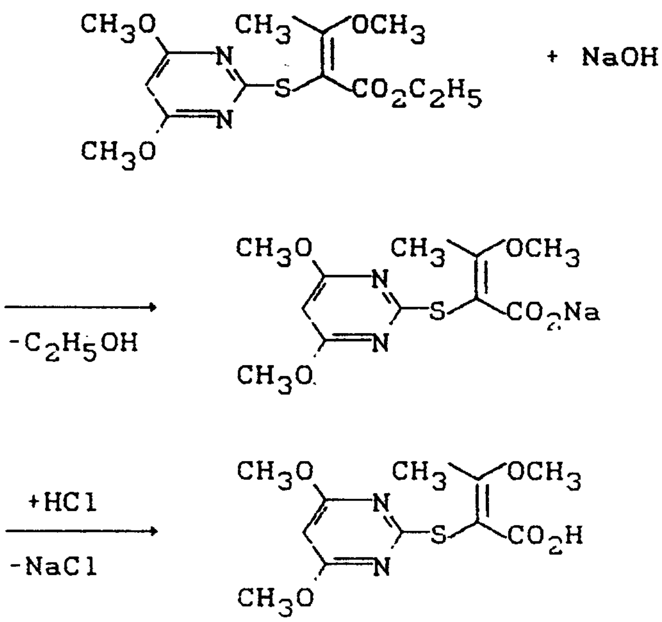


In the process (a), the starting compounds of the formula (II) mean compounds based on the above definitions of $R^2$, $R^3$ and $R^4$, preferably substituents based on the above preferred definitions of $R^2$, $R^3$ and $R^4$.

The compounds of the formula (II) can be obtained in the same way as described in Japanese Laid-open Patent Application No. 135963/1991. 2-(4,6-dimethoxy-2-pyrimidinylthio)acetic acid methyl ester, which is a representative example of the compounds of the general formula (II), is a known compound.

As specific example of the formula (II), there may be mentioned;
methyl, ethyl, n-propyl, n-butyl, and n-pentyl esters of 2-(4,6-dimethoxy-2-pyrimidinylthio)acetic acid.

The compounds of the formula (II) can be obtained when
(e) compounds of the formula (VI)

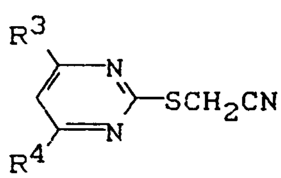 (VI)

wherein $R^3$ and $R^4$ have the same meanings as mentioned above,
are reacted with compounds of the formula (VII)

$R^2$-OH (VII)

wherein $R^2$ has the same meanings as mentioned above,
if appropriate, in the presence of inert solvents, and if appropriate, in the presence of an acid catalyst.

The compounds of the formula (VI) are novel and can be obtained when
(f) the compounds of the formula (IV) are reacted with compounds of the formula (VIII)

Y-$CH_2$CN (VIII)

wherein Y represents halogen or methylsulfonyloxy,
if appropriate, in the presence of inert solvents and, if appropriate, in the presence of an acid binder, or
(g) compounds of the formula (IX)

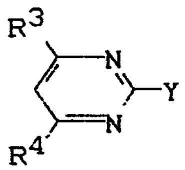

(IX)

wherein $R^3$, $R^4$ and Y have the same meanings as mentioned above,

are reacted with mercaptoacetonitrile,
if appropriate, in the presence of inert solvents and, if appropriate, in the presence of an acid binder.

The compounds of the formula (IV), (VIII) and (IX), and mercaptoacetonitrile are known compounds in the field of organic chemistry.

Further, the 2-mercaptopyrimidines represented by the formula (IV) can be obtained by reacting the corresponding halogen-substituted pyrimidines with hydrogen sulfide according to a method disclosed, for example, in Japanese Laid-Open Patent Application Nos, 111083/1975 and 125285/1976.

However, it should be noted that, according to such known methods, 2-mercaptopyrimidines cannot be obtained in a high yield.

It has also been found that the compounds of the formula (IV) can be obtained in a high yield when
(h) the 2-methanesulfonylpyrimdines of the formula (X)

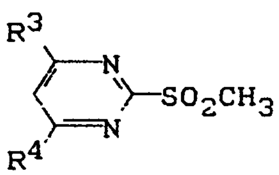

(X)

wherein $R^3$ and $R^4$ have the meanings as mentioned above,
are reacted with a thiolizing agent, in the presence of an inert solvent.

In the process (a), as specific examples of the formylating agents, there may be mentioned:
methyl formate, methyl orthoformate and the like.

In the process (b), the starting compounds of the formula (Ia) can be obtained by the process (a).

In the process (b), the starting compounds of the formula (III) mean compounds based on the above definitions of $R^6$ and X. Preferably, $R^6$ has the same preferably meanings as $R^1$, except hydrogen, and X represents chlorine, bromine, methanesulfonyloxy or p-toluenesulfonyloxy.

The alkylating agents also include, for example, dimethyl sulfate and diethyl sulfate, in addition to the compounds represented by the formula (III).

The compounds of the formula (III) are known compounds in the field of organic chemistry. As specific examples of the compounds of the formula (III), there may be mentioned:
methyl iodide, ethyl bromide, benzyl chloride and allyl chloride.

In the process (c), the starting compounds of the formula (V) mean compounds based on the above definitions of $R^2$ and $R^7$. Preferably, $R^2$ provides those based on the above substituents preferred definitions of $R^2$, and $R^7$ has the same preferable meaning as $R^5$, except hydrogen.

The compounds of the formula (V) are known compounds in the field of organic chemistry. As specific examples of the compounds of the formula (V), there may be mentioned:
2-chloroacetoacetic acid ethyl ester, 2-chloroacetoacetic acid methyl and 2-chlorobenzoylacetic acid ethyl ester.

In the process (d), the starting compounds of the formula (Ib) can be obtained by those processes (a), (b) and (c) as mentioned above.

In carrying out the process (a) as mentioned above, use may be made, as suitable diluent, of any inert solvent.

Examples of such diluents are aliphatic, cycloaliphatic and aromatic, optionally chlorinated, hydrocarbons such as pentane, hexane, cyclohexane, petroleum ether, ligroin, benzene, toluene, xylene and the like: ethers such as diethyl ether, methyl ethyl ether, diisopropyl ether, dibutyl ether, dioxane, tetrahydrofurane (THF) and the like: ketones such as acetone, methylethyl ketone (MEK), methyl-iso-propyl ketone, methyl-iso-butyl ketone (MIBK) and the like: nitriles such as acetonitrile, propionitrile and the like: alcohols such as

methanol, ethanol, isopropanol, butanol, ethylene glycol and the like: esters such as ethyl acetate, amyl acetate and the like, acid amides such as dimethyl formamide (DMF), dimethyl acetamide (DMA) and the like: sulfones and sulfoxides such as dimethyl sulfoxide (DMSO), sulfolane and the like: and bases such as pyridine.

In the above mentioned process (a), the reaction temperature can be varied within a substantially wide range. In general, the reaction is carried out at a temperature from -80 °C to about 150 °C, preferably from -10 °C to about 100 °C.

Further, the reaction is carried out under normal pressure, although it is also possible to employ a higher or reduced pressure.

When the above mentioned process (a) according to the present invention is carried out, use is made, for example, about 1.0 to 2.0 mols of the formylating agent per 1 mol of the compounds represented by the general formula (II) to obtain the desired compounds.

In carrying out the above mentioned process (b), use may be made, as suitable diluent, of any inert solvents, including, for instance, those shown in the paragraph directed to the explanation of the process (a).

In the above mentioned process (b), the reaction temperature can be varied within a substantially wide range. In general, the reaction is carried out at a temperature from about -30 °C to about 150 °C, preferably from -5 °C to about 80 °C.

Further, the reaction is carried out under normal pressure, although it is also possible to employ a higher or reduced pressure.

When the above mentioned process (b) according to the present invention is carried out, use is made, for instance, 1.0 to 1.5 mol of the compounds of the formula (III) or alkylating agents in a diluent per 1 mol of the compounds of the formula (Ia) to obtain the desired compounds.

In carrying out the above mentioned process (c), use may be made, as suitable diluent, of any inert solvent.

Examples of such diluents are water; aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, chlorobenzene, dichlorobenzene and the like; ethers such as diethyl ether, methyl ethyl ether, diisopropyl ether, dibutyl ether, dioxane, dimethoxyethane (DME), tetrahydrofurane (THF), diethyleneglycol dimethylether (DGM), and the like; ketones; acetone, methylethyl ketone (MEK), methyl-iso-propyl ketone, methy-isobutyl ketone, and the like; nitriles such as acetonitrile, propionitrile acrylonitrile and the like; alcohols; methanol, ethanol, isopropanol, butanol, ethylene glycol and the like; esters such as ethyl acetate, amyl acetate and the like; acid amides such as dimethyl formamide (DMF), dimethyl acetamide (DMA), N-methylpyrrolidone, 1,3-dimethyl-2-imidazolidinone, hexamethylphosphoric triamide (HMPA) and the like; sulfones and sulfoxides such as dimethyl sulfoxide (DMSO), sulfolane and the like; and base such as pyridine.

The process (c) according to the invention is carried out preferably in the presence of acid binder. As example of such acid binder may be mentioned:
inorganic bases including hydroxide, carbonate, bicarbonate, alcoholate of alkali metals such as, for example, sodium hydrogen carbonate, potassium hydrogen carbonate, sodium carbonate, potassium carbonate, organic bases including tertiary amines, dialkylaminoanilines, and pyridines such as, for example, triathylamine, tributylamine, 1,1,4,4-tetra-methylenadiamine (TMEDA), N,N-dimethylaniline, N,N-diethylaniline, pyridine, 4-dimethylamino pyridine (DMAP), 1,4-diazabicyclo[2,2,2]octane (DABCO), 1,8-diazabicyclo[5,4,0]-undec-7-ene (DBU), organic lithium compounds such as methyl lithium, n-butyl lithium, sec-butyl lithium, tert-butyl lithium, phenyl lithium, dimethyl copper lithium, lithium diisopropyl amide, lithium cyclohexylisopropyl amide, lithium dicylohexyl amide, n-butyl lithium•DABCO, n-butyl lithium•DBU, n-butyl lithium•TMEDA and the like.

In the above mentioned process (c), the reaction temperature can be varied within a substantially wide range. In general, the reaction is carried out at a temperature from -60 °C to about 150 °C, preferably from -10 °C to about 80 °C.

Further, the reaction is carried out under normal pressure, although it is also possible to employ a higher or reduced pressure.

When the above mentioned process (c) according to the present invention is carried out, use is made, for example, 1.0 to 1.5 mol of the compounds of the formula (V) in a diluent such as acetonitrile per 1.0 mol of the compounds of the formula (IV) in the presence of an acid binder to obtain the desired compounds.

In carrying out the above mentioned process (d), use may be made, as suitable diluent, of any inert solvent.

Examples of such diluents are water, ethers such as diethyl ether, methyl ethyl ether, diisopropyl ether, dibutyl ether, dioxane, dimethoxyethane (DME), tetrahydrofurane (THF), diethyleneglycol dimethylether (DGM), and the like; ketones; acetone, methylethyl ketone (MEK), methyl-isopropyl ketone, methyl-iso-butyl ketone, and the like; nitriles such as acetonitrile, propionitrile acrylonitrile and the like; alcohols such as methanol, ethanol, isopropanol, butanol, ethylene glycol and the like; esters such as ethyl acetate, amyl acetate and the like; acid amides such as dimethyl formamide (DMF), dimethyl acetamide (DMA), N-methyl-pyrrolidone, 1,3-dimethyl-2-imidazolidinone, hexamethyl-phosphoric triamide (HMPA) and the like; sulfones and sulfoxides such as dimethyl sulfoxide (DMSO), sulfolane and the like.

The process (d) according to the invention is carried out preferably in the presence of acid binder and as example of such acid binder may be mentioned; inorganic bases including hydroxide, carbonate, bicarbonate and alcolate of alkali metals, such as sodium hydrogen carbonate, potassium hydrogen carbonate, sodium carbonate, potassium carbonate, lithium hydroxide, sodium hydroxide, potassium hydroxide, calcium hydroxide and the like, inorganic amides of alkali metals such as lithium amide, sodium amide, potassium amide and the like, and organic bases including tertiary amines, dialkylaminoanilines and pyridines such as, triethylamine, 1,1,4,4-tetramethylene-diamine (TMEDA), N,N-dimethylaniline, N,N-diethylaniline, pyridine, 4-dimethylamino pyridine (DMAP), 1,4-diazabicyclo-[2,2,2] octane (DABCO), 1,8-diazabicyclo[5,4,0]-undec-7-ene (DBU) and the like. Furthermore, organic lithium compounds such as methyl lithium, n-butyl lithium, secbutyl lithium, tert-butyl lithium, phenyl lithium, dimethyl copper lithium, lithium diisopropyl amide, lithium cyclohexylisopropyl amide, lithium dicyclohexyl amide, n-butyl lithium•DABCO, n-butyl lithium•DBU, n-butyl lithium•TMEDA and the like.

In the above mentioned process (d), the reaction temperature can be varied within a substantially wide range. In general, the reaction is carried out at a temperature from -40 °C to about 100 °C, preferably from 0 °C to about 50 °C

Further, the reaction is carried out under normal pressure, although it is also possible to employ a higher or reduced pressure.

When the above mentioned process (d) according to the present invention is carried out, use is made, for example, 1 mol of the compounds of the formula (Ib), are subjected to a hydrolysis reaction in a diluent such as water in the presence of 1 to 2 mol of a base to obtain the desired compounds.

The active compounds according to the invention can be used as defoliants, desiccants, agents for destroying broad-leaved plants and, especially, as weedkillers. By weeds, in the broadest sense, there are to be understood all plants which grow in locations where they are undesired. Whether the substances according to the invention act as total or selective herbicides depends essentially on the amount used.

The active compounds according to the invention can be used, for example, in connection with the following plants:

Dicotyledon weeds of the genera: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver and Centaurea.

Dicotyledon cultures of the genera: Gossypium, Glycine, Beta, Daucus, Phaseolus, Pisum, Solanum, Linum, Ipomoea, Vicia, Nicotiana, Lycopersicon, Arachis, Brassica,

Monocotyledon weeds of the genera: Echinochloa, Setaria, Panicum, Digitaria, Phleum, Poa, Festuca, Eleusine, Brachiaria, Lolium, Bromus, Avena, Cyperus, Sorghum, Agropyron, Gynodon, Monochoria, Fimbristylis, Sagittaria, Eleocharis, Scirpus, Paspalum, Ischaemum, Sphenoclea, Dactyloctenium, Agrostis, Alopecurus and Apera.

Monocotyledon cultures of the genera: Oryza, Zea, Triticum, Hordeum, Avena, Secale, Sorghum, Panicum, Saccharum, Ananas, Asparagus and Allium.

However, the use of the active compounds according to the invention is in no way restrictred to these genera, but also extends in the same manner to other plants.

The compounds are suitable, depending on the concentration, for the total combating of weeds, for example on industrial terrain and rail tracks, and on paths and squares with or without tree plantings.

Equally, the compounds can be employed for combating weeds in perennial cultures, for example afforestations, decorative tree plantings, orchards, vineyards, citrus groves, nut orchards, banana plantations, coffee plantations, tea plantations, rubber plantations, oil palm plantations, cocoa plantations, soft fruit plantings and hopfields, and for the selective combating of weeds in annual cultures.

The active compounds can be converted into the customary formulations, such as solutions, emulsions, wettable powders, suspensions, powders, foams, pastes, granules, aerosols, natural and synthetic materials impregnated with active compound, very fine capsules in polymeric substances, coating compositions for use on seed, and formulations used with burning equipment, such as fumigating cartridges, fumigating cans

and fumigating coils, as well as ULV cold mist and warm mist formulations.

These formulations may be produced in known manner, for example by mixing the active compounds with extenders, that is to say liquid or liquefied gaseous or solid diluents or carriers, optionally with the use of surface-active agents, that is to say emulsifying agents and/or dispersing agents and/or foam-forming agents. In the case of the use of water as an extender, organic solvents can, for example, also be used as auxiliary solvents.

As liquid solvents diluents or carriers, there are suitable in the main, aromatic hydrocarbons, such as xylene, toluene or alkyl naphthalenes, chlorinated aromatic or chlorinated aliphatic hydrocarbons, such as chlorobenzenes, chloroethylenes or methylene chloride, aliphatic hydrocarbons, such as cyclohexane or paraffins, for example mineral oil fractions, alcohols, such as butanol or glycol as well as their ethers and esters, ketones, such as acetone, methyl ethyl ketone, methyl isobutyl ketone or cyclohexanone, or strongly polar solvents, such as dimethylformamide and dimethylsulphoxide, as well as water.

By liquefied gaseous diluents or carriers are meant liquids which would be gaseous at normal temperature and under normal pressure, for example aerosol propellants, such as halogenated hydrocarbons as well as butane, propane, nitrogen and carbon dioxide.

As solid carriers there may be used ground natural minerals, such as kaolins, clays, talc, chalk, quartz, attapulgite, montmorillonite or diatomaceous earth, and ground synthetic minerals, such as highly-dispersed silicic acid, alumina and silicates. As solid carriers for granules there may be used crushed and fractionated natural rocks such as calcite, marble, pumice, sepiolite and dolomite, as well as synthetic granules of inorganic and organic meals, and granules or organic material such as sawdust, coconut shells, maize cobs and tobacco stalks.

As emulsifying and/or foam-forming agents there may be used non-ionic and anionic emulsifiers, such as polyoxyethylene-acid esters, polyoxyethylene-alcohol ethers, for example alkylaryl polyglycol ethers, alkyl sulphonates, alkyl sulphates, aryl sulphonates as well as albumin hydrolysis products.

Dispersing agents include, for example, lignin sulphite waste liquors and methylcellulose.

Adhesives such as carboxymethylcellulose and natural and synthetic polymers in the form of powder, granules or latices, such as gum arabic, polyvinyl alcohol and polyvinyl acetate, can be used in the formulation.

It is possible to use colorants such as inorganic pigments, for example iron oxide, titanium oxide and Prussian Blue, and organic dyestuffs, such as alizarin dyestuffs, azo dyestuffs or metal phthalocyanine dyestuffs, and trace nutrients, such as salts of iron, manganese boron, copper, cobalt, molybdenum and zinc. The formulations in general contain from 0.1 to 95 per cent by weight of active compound, preferably from 0.5 to 90 per cent by weight.

The active compounds according to the invention, as such or in the form of their formulations, can also be used, for combating weeds, as mixtures with known herbicides, finished formulations or tank mixes being possible.

Mixtures with other known active compounds, such as herbicides, fungicides, insecticides, acaricides, nematicides, bird repellents, plant nutrients and agents which improve soil structure, are also possible.

The active compounds can be used as such, in the form of their formulations or in the use forms prepared therefrom by further dilution, such as ready-to-use solutions, suspensions, emulsions, powders, pastes and granules. They are used in the customary manner, for example by watering, spraying, atomizing or scattering.

The active compounds according to the invention can be applied either before or after emergence of the plants.

They can also be incorporated into the soil before sowing. They are used, in particular, after emergence of the plants.

The amount of active compound used can vary within a substantial range. It depends essentially on the nature of the desired effect. In general, the amounts used are between 0.01 and 10 kg of active compound per hectare of soil surface, preferably between 0.05 and 5 kg per ha.

The preparation and use of the active compounds according to the invention can be seen from the following examples.

Preparation Examples:

Example 1

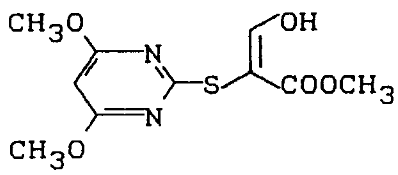

Diisopropylamine (20.0 g) was mixed with anhydrous tetrahydrofurane (200 ml) under nitrogen gas atmosphere. To the resulting mixture was dropwise added a solution (125 ml) of n-butyl lithium (1.6 mol) in hexane at a temperature from -78 to -60 °C. The mixture was stirred for 10 minutes, and thereafter a solution of 2-(4,6-dimethoxy-2-pyrimidinylthio)acetic acid methyl ester (25 g) in anhydrous tetrahydrofuran (200 ml) was dropwise added to the reaction mixture while maintaining the above-mentioned temperature. After the completion of this addition, the reaction mixture was stirred for 30 minutes. Thereafter, methyl formate (8.7 g) was dropwise added to the reaction mixture at a temperature between -78 and -60 °C. Then the temperature of the reaction mixture was slowly raised to room temperature, and thereafter the reaction mixture was further stirred for 10 hours. The solvent was distilled off under reduced pressure, and water (500 ml) was added. The resultant mixture was washed several times with ethyl acetate, and the aqueous layer was acidified with 2N-hydrochloric acid. Then an extraction operation was carried out by using ethyl acetate. The organic layer was dried over anhydrous sodium sulfate, and the solvent was distilled off under reduced pressure to give 3-hydroxy-2-(4,6-dimethoxy-2-pyrimidinylthio)acrylic acid methyl ester (14.2 g).
mp. 68 - 72 °C

Example 2

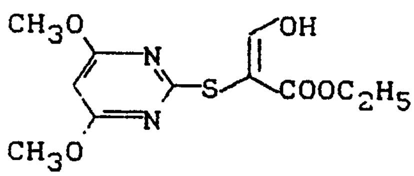

A mixture of 2-(4,6-dimethoxy-2-pyrimidinyl-thio)acetic acid ethyl ester (10 g), methyl formate (4.5 g) and anhydrous tetrahydrofuran (100 ml) was dropwise added at room temperature to a mixture of potassium tertbutoxide (4.8 g) and anhydrous tetrahydrofuran (100 ml). After the completion of this addition, the reaction mixture was heated under reflux for 30 minutes. Then the reaction mixture was allowed to cool to room temperature, and thereafter the solvent was distilled off under reduced pressure. The resulting residue was admixed with water (200 ml), and washed several times with ethyl acetate. The aqueous layer was acidified with 2N-hydrochloric acid, and then an extraction operation was carried out by using ethyl acetate. The organic layer was dried over anhydrous sodium sulfate, and the solvent was distilled off under reduced pressure to give 3-hydroxy-2-(4,6-dimethoxy-2-pyrimidinylthio)acrylic acid ester (6.5 g).
$n_D^{20}$ 1.5420.

Example 3

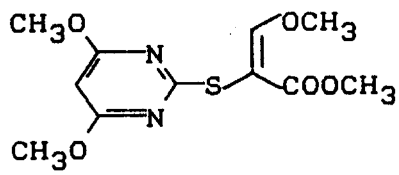

Dimethyl sulfate (2.8 g) was dropwise added at 0°C to a mixture of 3-hydroxy-2-(4,6-dimethoxy-2-pyrimidinylthio)-4-acrylic acid methyl ester (3 g), anhydrous potassium carbonate (6.1 g) and anhydrous dimethyl formamide (50 ml). After the completion of this addition, the temperature of the reaction mixture was slowly raised to room temperature, and then the reaction mixture was stirred for further 48 hours. Water (200 ml) was added, and an extraction operation was carried out by using ethyl acetate. The organic layer was dried over anhydrous sodium sulfate, and the solvent was distilled off under reduced pressure. A crude product thus obtained was refined by means of a column chromatography, wherein use was made of hexane/ethyl acetate as eluent, to give 3-methoxy-2-(4,6-dimethoxy-2-pyrimidinylthio) acrylic acid ethyl ester (2.9 g).
mp. 117.5 - 119.5°C.

Example 4

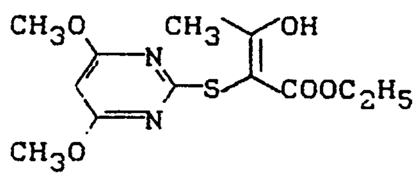

A mixture of 4,6-dimethoxy-2-mercaptopyrimidin (5.2 g), 2-chloroacetoacetic acid ethyl ester (5.2 g) and potassium carbonate (5 g) in acetonitrile was stirred for 15 hours at room temperature. An insoluble matter was removed by filtration, and the acetonitrile was evaporated under reduced pressure. The residue was extracted with hexane. The hexane thus obtained was refined by means of a column chromatography, wherein use was made of hexane/ethyl acetate as eluent, to give 2-(4,6-dimethoxy-2-pyrimidinyl-thio)-acetoacetic acid ethyl ester (5.1 g).
$n_D^{20}$ 1.5378

Example 5

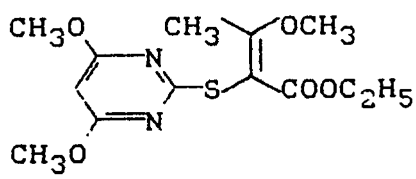

Dimethyl sulfate (2.8 g) was dropwise added at 0°C to a mixture of 2-(4,6-dimethoxy-2-pyrimidinylthio)-acetoacetic acid ethyl ester (2.8 g) and tert-butoxide (1.2 g). The reaction mixture was stirred at room temperature for further 15 hours. Water was added, and an extraction operation was carried out by using ethyl acetate. The organic layer was dried over anhydrous sodium sulfate, and the solvent was distilled off under reduced pressure. A crude product thus obtained was refined by means of a column chromatography

to give 3-methoxy-2-(4,6-dimethoxy-2-pyrimidinylthio)crotonic acid ethyl ester (1.9 g).
mp. 101 - 103.5 °C
Following table 2 shows the compounds of the invention which may be obtained by the same method as above preparation examples 1 to 5, together with the compounds that were prepared in Examples 1 to 5.

Table 2

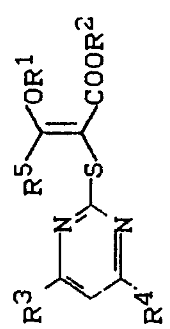

| Compound No. | $R^1$ | $R^2$ | $R^3$ | $R^4$ | $R^5$ | |
|---|---|---|---|---|---|---|
| 1 | H | $CH_3$ | $OCH_3$ | $OCH_3$ | H | mp. 86-72° C |
| 2 | $CH_3$ | $CH_3$ | $OCH_3$ | $OCH_3$ | H | mp. 117.5-119.5° C |
| 3 | $C_2H_5$ | $CH_3$ | $OCH_3$ | $OCH_3$ | H | mp. 89.5-90° C |
| 4 | $C_3H_7$-iso | $CH_3$ | $OCH_3$ | $OCH_3$ | H | mp. 99-100.5° C |
| 5 | $CH_2$- 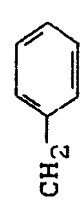 | $CH_3$ | $OCH_3$ | $OCH_3$ | H | mp. 103.5-105° C |
| 6 | $CH_2CH=CH_2$ | $CH_3$ | $OCH_3$ | $OCH_3$ | H | mp. 57.5-62° C |
| 7 | H | $C_2H_5$ | $OCH_3$ | $OCH_3$ | H | $n_D^{20}$ 1.5420 |
| 8 | $CH_3$ | $C_2H_5$ | $OCH_3$ | $OCH_3$ | H | $n_D^{20}$ 1.5461 |
| 9 | $CH_2CH_2Br$ | $CH_3$ | $OCH_3$ | $OCH_3$ | H | $n_D^{20}$ 1.5661 |

Table 2 (continued)

| Compound No. | $R^1$ | $R^2$ | $R^3$ | $R^4$ | $R^5$ | |
|---|---|---|---|---|---|---|
| 10 | $CH_2C{\equiv}CH$ | $CH_3$ | $OCH_3$ | $OCH_3$ | H | $n_D^{20}$ 1.5189 |
| 11 | H | $CH_3$ | $CH_3$ | $CH_3$ | H | oil |
| 12 | $CH_3$ | $CH_3$ | $CH_3$ | $CH_3$ | H | oil |
| 13 | H | $CH_3$ | $OCH_3$ | $OCH_3$ | $C_2H_5$ | $n_D^{20}$ 1.5299 |
| 14 | H | $C_2H_5$ | $OCH_3$ | $OCH_3$ | $CH_2CH_2CH_3$ | $n_D^{20}$ 1.5257 |
| 15 | H | $CH_3$ | $OCH_3$ | $OCH_3$ | $CH(CH_3)_2$ | $n_D^{20}$ 1.5262 |
| 16 | H | $C_2H_5$ | $OCH_3$ | $OCH_3$ | $CH_3$ | $n_D^{20}$ 1.5378 |
| 17 | H | $C_2H_5$ | $OCH_3$ | $OCH_3$ | $CH_2OCH_3$ | mp. 61.5-64° C |
| 18 | H | $C_2H_5$ | $OCH_3$ | $OCH_3$ | 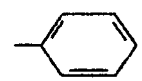 | mp. 78-81° C |

<u>Table 2</u> (continued)

| Compound No. | $R^1$ | $R^2$ | $R^3$ | $R^4$ | $R^5$ | |
|---|---|---|---|---|---|---|
| 19 | $CH_3$ | $C_2H_5$ | $OCH_3$ | $OCH_3$ | $CH_3$ | mp. 101-103.5° C |
| 20 | $C_2H_5$ | $C_2H_5$ | $OCH_3$ | $OCH_3$ | $CH_3$ | mp. 111.5-113.5° C |
| 21 | $CH_2CH_2CH_3$ | $C_2H_5$ | $OCH_3$ | $OCH_3$ | $CH_3$ | $n_D^{20}$ 1.4994 |
| 22 | $CH_2CH_2Br$ | $C_2H_5$ | $OCH_3$ | $OCH_3$ | $CH_3$ | mp. 78-82.5° C |
| 23 | $CH_2$ 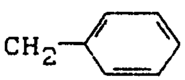 | $C_2H_5$ | $OCH_3$ | $OCH_3$ | $CH_3$ | $n_D^{20}$ 1.5548 |
| 24 | $CH_3$ | $C_2H_5$ | $OCH_3$ | $OCH_3$ | $CH_2CH_2CH_3$ | $n_D^{20}$ 1.5462 |
| 25 | $CH_3$ | $C_2H_5$ | $OCH_3$ | $OCH_3$ | 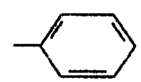 | $n_D^{20}$ 1.5779 |
| 26 | $-CH_2$ 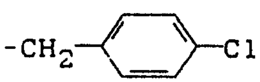 Cl | $CH_3$ | $OCH_3$ | $OCH_3$ | H | mp. 107-110° C |
| 27 | $-CH_2$ 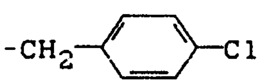 Cl | $C_2H_5$ | $OCH_3$ | $OCH_3$ | $CH_3$ | $n_D^{20}$ 1.5457 |

Table 2 (continued)

| Compound No. | $R^1$ | $R^2$ | $R^3$ | $R^4$ | $R^5$ | |
|---|---|---|---|---|---|---|
| 28 | $CH_3$ | $CH_3$ | $OCH_3$ | $OCH_3$ | $CH(CH_3)_2$ | $n_D^{20}$ 1.5279 |
| 29 | H | $CH_3$ | $OCH_3$ | $OCH_3$ | $C_4H_9$-t | mp. 58-61° C |
| 30 | H | $CH_3$ | $OCH_3$ | $OCH_3$ | 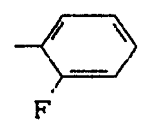 | $n_D^{20}$ 1.5435 |

Example IV-1

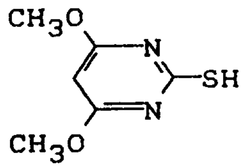

A solution (250 ml) of sodium hydrosulfide•$nH_2O$ (purity: about 70%; 35.0 g) in methanol was added at room temperature under stirring to a solution of 4,6-dimethyl-2-methylsulfonyl pyrimidine (50.0 g in acetonitrile (400 ml). The reaction mixture was stirred at room temperature for 3 hours, and then the solvent was distilled off under reduced pressure. The resultant residue was admixed with water, and insoluble matters were removed by filtration. The solution thus obtained was acidified with concentrated hydrochloric acid to crystallize out the desired compound, which was then separated by filtration, washed with water and dried to give 4,6-dimethoxy-2-mercaptopyrimidine (37.5 g). The purity of this product was 98%.
Yield: 95%. mp. >300 ° C

Reference Example 1

Preparation of 4,6-dimethoxy-2-mercaptopyrimidine

2-Chloro-4,6-dimethoxypyrimidine (2.0 g) and sodium hydosulfide (2.30 g) were dissolved in methanol (40 ml), and the resulting solution was heated under reflux for 12 hours.

After cooling, the solvent was distilled off under reduced pressure, and water was added. Thereafter, an extraction operation was carried out by using ether. The ether layer was dried over anhydrous magnesium sulfate, and then the solvent was distilled off under reduced pressure to give 2-chloro-4,6-dimethoxypyrimidine (1.30 g). mp. 101-102 ° C.

Thereafter, the aqueous layer was acidified with concentrated hydrochloric acid to pH 2, and the resultant precipitate was separated by filtration, and dried to give 4,6-dimethoxy-2-mercaptopyrimidine (0.48 g).
Yield: 24%

Reference Example 2

Preparation of 4,6-dimethoxy-2-mercaptopyrimidine

2-Chloro-4,6-dimethoxypyrimidine (2.0 g) and sodium hydrosulfide (2.30 g) were dissolved in methanol, and the resulting solution was stirred at room temperature for 3 hours. The solvent was distilled off under reduced pressure, and the residue thus obtained was admixed with water. An extraction operation was carried out by using ether. The ether layer was dried over anhydrous magnesium sulfate, and then the solvent was distilled off under reduced pressure to give 2-chloro-4,6-dimethoxypyrimidine (1.95 g). mp. 101-102 ° C.

The aqueous layer was acidified with concentrated hydrochloric acid to pH 2, and the crystalline material thus formed was separated by filtration, and dried to give 4,6-dimethoxy-2-mercaptopyrimidine (0.01 g).
Yield: 0.5%.

Example IV-2

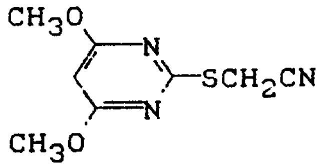

A solution of 4,6-dimethoxy-2-mercaptopyrimidine (10.0 g) and chloroacetonitrile (4.4 g) in acetonitrile (100 ml) was admixed with potassium carbonate (10.0 g), and heated under reflux for 2 hours. After cooling, unsolved matters were removed by filtration, and the acetonitrile was distilled off under reduced pressure. The resulting residue was dissolved in toluene, and insoluble matters were removed by filtration. Then the toluene was distilled off under reduced pressure, and the residue thus obtained was treated with an ether/hexane mixture to crystallize out and to give 2-(4,6-dimethoxy-2-pyrimidinylthio)acetonitrile (11.3 g). mp. 102.5-103.5 ° C.

Referential Synthesis Example 1 synthesis of intermediate

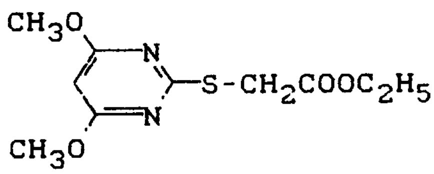

1-(1):
A mixture of ethyl bromoacetate (167 g), 4,6-dimethoxy-2-mercaptopyrimidine (172 g), anhydrous potassium carbonate (152 g) and acetonitrile (1 liter) was heated under reflux for 5 hours. After cooling, the solvent was distilled off under reduced pressure, water (1 liter) was added, and an extraction operation was effected three times by using ethyl acetate. The organic layer was dried over anhydrous sodium sulfate, and the solvent was distilled off under reduced pressure. A crude product was obtained and recrystallized from diisopropyl ether, to give 2-(4,6-dimethoxy-2-pyrimidinyloxythio) acetic acid ethyl ester (245 g). mp. 59-61 ° C
1-(2):
A mixture of ethyl thioglycolate (120 g), 4,6-dimethoxy-2-methylsulfonylpyrimidine (214 g), anhydrous potassium carbonate (166 g) and acetonitrile (1 liter) was heated under reflux for 5 hours. After cooling, the solvent was distilled off under reduced pressure, water (1 liter) was added, and an extraction operation was carried out three times by using ethyl acetate. The organic layer was dried over anhydrous sodium sulfate, and the solvent was distilled off under reduced pressure. A crude product was obtained and recrystallized from diisopropyl ether, to give 2-(4,6-dimethoxy-2-pyrimidinylthio) acetic acid ethyl ester (232 g).
1-(3):
A solution of 2-(4,6-dimethoxy-2-pyrimidinylthio)acetonitrile (1.1 g) in ethanol (15 ml) was cooled with ice, and admixed with ethanol saturated with hydrochloric acid (10 ml).

After standing overnight at room temperature, the reaction mixture was admixed with water (0.1 g), and stirred for 3 hours. The ethanol was distilled off under reduced pressure, toluene was added, and the resulting mixture was washed with water. The toluene layer was dried, and the solvent was distilled off to give 2-(4,6-dimethoxy-2-pyrimidinylthio)acetic acid ethyl ester (1.1 g).

Comparative Compound

C-1

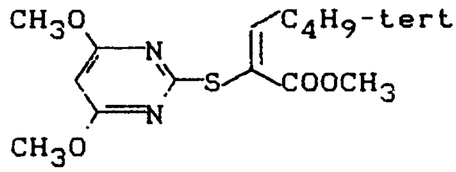

(disclosed in Japanese Laid-Open Patent Application No. 135964/1991)

Test Example 1

Pre-emergence soil treatment test on upland weeds

Formuation of Active Compounds

Carrier: 5 parts by weight of acetone
Emulsifier: 1 part by weight of benzyloxy polyglycol ether

To produce a suitable formulation of each of the active compounds, 1 part by weight of the active compound was mixed with stated amount of carrier and with the stated amount of emulsifier, and the resulting emulsifiable concentrate was diluted with water to the desired concentration.

Test Procedures

In a greenhouse, a number of test pots each having an area of 120 $cm^2$ were charged with soil taken out from a cultivated field. Onto the soil surface in the respective test pots were sown the seeds of barnyard grass (Echinochloa crus-gall), bottleglass (Setaria viridis), Amaranthus (Amaranthus viridis) and polygonum (polygonum convolvulus), respectively, followed by soil covering thereon. Thereafter, predetermined dosages of the active compound formulation mentioned above were uniformly sprayed onto the soil surface of the respective test pots.

Four weeks after the application of the active compound formulations, the degrees of herbicidal effect on the weeds were determined.

A clearly superior herbicidal activity combined with an equally good selectivity in crop plants, is shown in comparison to comparative compound C-1, in this test, for example by the compounds of following preparation examples: 8, 9, 19, 20, 21, 23, 24 and 25.

Test Example 2

Post-emergence foliage treatment on upland weeds

Test Procedures

In a greenhouse, a number of test pots having an area of 250 $cm^2$ were charged with soil taken out from a cultivated field. Seeds of barnyard grass (Echinochloa crus-gall), bottlegrass (Setaria viridis), Amaranthus (Amaranthus viridis) and polygonum (polygonum convolvulus) respectively, were sown onto the soil surface in the respective test pots, followed by cultivation for ten days. When the test weeds were generally in the two-leaves stage, predetermined dosages of the active compound formulations prepared as in example 3 mentioned above were uniformly sprayed onto the foliage portions of the test weeds in the respective test pots.

Three weeks after the application of the active compound formulations, the degrees of herbicidal effect on the weeds were determined.

A clearly superior herbicidal activity combined with an equally good selectivity in crop plants, is shown in comparison to comparison compound C-1, in this test, for example by the compounds of following preparation examples: 8, 9, 19, 20, 21, 23, 24 and 25.

## Claims

**1.** Pyrimidinylthioacrylic acid derivatives of the formula (I)

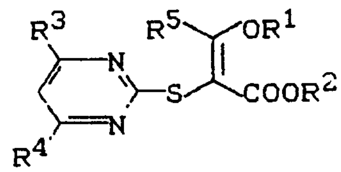

(I)

wherein

| | |
|---|---|
| $R^1$ | represents hydrogen or in each case optionally substituted $C_{1-6}$ alkyl, $C_{2-4}$ alkenyl or $C_{2-4}$ alkynyl, |
| $R^2$ | represents hydrogen or optionally substituted $C_{1-6}$ alkyl or alkali salts, |
| $R^3$ and $R^4$ | each independently represent $C_{1-4}$ alkyl, $C_{1-4}$ alkoxy, halogeno-$C_{1-4}$ alkyl, halogeno-$C_{1-4}$ alkoxy or halogen, and |
| $R^5$ | represents hydrogen, or in each case optionally substituted $C_{1-6}$ alkyl, phenyl, $C_{2-4}$ alkenyl, $C_{2-4}$ alkynyl, or a heterocyclic ring, or represents an alkali metal salt, alkali earth metal or organic ammonium salt of pyrimidinylthioacrylic acid derivatives of the formula (I). |

**2.** The compounds of the formula (I) according to Claim 1, wherein

| | |
|---|---|
| $R^1$ | represents hydrogen, $C_{1-6}$ alkyl which are unsubstituted or substituted by at least one substituent selected from the group consisting of halogen, $C_{1-4}$ alkoxy, $C_{1-4}$alkylthio, acyl, optionally substituted phenyl or 5- or 6-membered heterocyclic ring having one or two hetero atoms selected from the group consisting of nitrogen, sulfur and oxygen, |
| $R^1$ | further represents $C_{2-4}$ alkenyl $C_{2-4}$ halogenoalkenyl, $C_{2-4}$ alkynyl and $C_{2-4}$ halogenoalkynyl, |
| $R^2$ | represents hydrogen, $C_{1-4}$ alkyl, $C_{1-2}$ alkoxy-$C_{1-2}$ alkyl, benzyl or alkali salts, |
| $R^3$ and $R^4$ | each independently represent methyl, ethyl, methoxy, ethoxy, halogeno-$C_{1-2}$ alkyl, halogeno-$C_{1-2}$ alkoxy or chlorine, and |
| $R^5$ | represents hydrogen, optionally substituted $C_{1-6}$ alkyl, suitable substituents are selected from the group consting of halogen, $C_{1-4}$ alkoxy, $C_{1-4}$ alkylthio,acyl and phenyl which may be substituted, $R^5$ further represents phenyl which is unsubstituted or substituted at leat by one substituent selected from the group consisting of halogen, $C_{1-4}$ alkyl and $C_{1-4}$ alkoxy, $R^5$ further represents $C_3$ alkenyl, $C_3$ alkynyl or a 5- or 6-membered heterocyclic ring having one or two hetero atoms selected from the group consisting of nitrogen, sulfur and oxygen and which may be substituted. |

**3.** The compounds of the formula (I) according to Claim 1, wherein

| | |
|---|---|
| $R^1$ | represents hydrogen, optionally substituted $C_{1-4}$ alkyl, suitable substituents are selected from the group consisting of fluorine, chlorine, methoxy, ethoxy, methylthio, ethylthio, acetyl, phenyl which is unsubstituted or substituted by at least one substituent selected from the group consisting of halogen, $C_{1-4}$ alkyl and $C_{1-2}$ alkoxy, pyridyl, thiazolyl, $R^1$ further represents $C_{2-3}$ alkenyl, $C_{2-3}$ chloroalkenyl, $C_3$ alkynyl and $C_3$ chloroalkynyl, |
| $R^2$ | represents hydrogen, methyl ethyl, benzyl or alkali salts, |
| $R^3$ and $R^4$ | represents methoxy, and |
| $R^5$ | represents hydrogen, optionally substituted $C_{1-4}$ alkyl, suitable substituents are selected from the group consisting of fluorine, chlorine, methoxy, ethoxy, methylthio and ethylthio or $R^5$ further represents phenyl which is unsubstituted or substituted by at least one substituent selected from the group consisting of fluorine, chlorine, methyl and methoxy, or $R^5$ further represents allyl, propargyl, pyridyl, thienyl, or furyl. |

**4.** Process for the preparation of pyrimidinylthioacrylic acid derivatives of the formula (I)

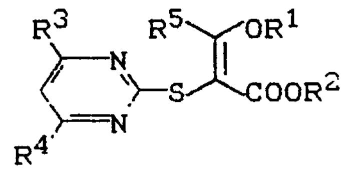

(I)

wherein

| | |
|---|---|
| $R^1$ | represents hydrogen or in each case optionally substituted $C_{1-6}$ alkyl, $C_{2-4}$ alkenyl or $C_{2-4}$ alkynyl, |
| $R^2$ | represents hydrogen or optionally substituted $C_{1-6}$ alkyl, benzyl or alkali salts, |
| $R^3$ and $R^4$ | each independently represent $C_{1-4}$ alkyl, $C_{1-4}$ alkoxy, halogeno-$C_{1-4}$ alkyl, halogeno-$C_{1-4}$ alkoxy or halogen, and |
| $R^5$ | represents hydrogen, or in each case optionally substituted $C_{1-6}$ alkyl, phenyl, $C_{2-4}$ alkenyl, $C_{2-4}$ alkynyl, or a heterocyclic ring, or represents an alkali metal salt, alkali earth metal or organic ammonium salt of pyrimidinylthioacrylic acid derivatives of the formula (I), |

characterized in that

(a) in the case where $R^1$ and $R^5$ represent hydrogen: compounds of the formula (II)

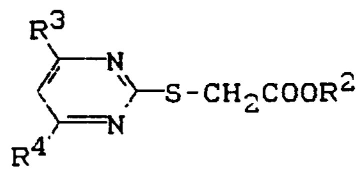

(II)

wherein $R^2$, $R^3$ and $R^4$ represent the same meanings as mentioned above, are reacted with a formylating agent, in the presence of inert solvents,

or

(b) in the case where $R^1$ has the meanings mentioned above, except hydrogen: compounds of the formula (Ia)

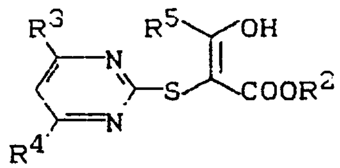

(Ia)

wherein $R^2$, $R^3$, $R^4$ and $R^5$ represent the same meanings as mentioned above, are reacted with compounds of the formula (III)

$R^6$ - X (III)

wherein $R^6$ has the meanings as $R^1$, except hydrogen, and X represents -O-$SO_2$-M, in which M represents alkyl or aryl, in the presence of inert solvents,

or

(c) in the case where $R^1$ represents hydrogen, and $R^5$ has the meanings mentioned above, except hydrogen: compounds of the formula (IV)

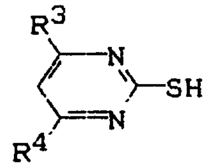

(IV)

wherein $R^2$ and $R^3$ have the same meanings as mentioned above, are reacted with compounds of the formula (V)

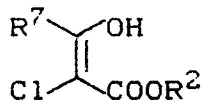

(V)

wherein $R^2$ has the same meaning as mentioned above and $R^7$ has the same meaning as $R^5$ except hydrogen, in the presence of inert solvents and if appropriate, in the presence of acid binder,

or

(d) in the case where $R^2$ represents hydrogen: compounds of the formula (Ib)

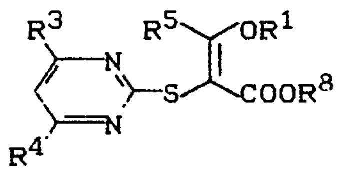

(Ib)

wherein $R^1$, $R^3$, $R^4$ and $R^5$ have the same meanings as mentioned above, and $R^8$ represents optionally substituted $C_{1-6}$ alkyl, are hydrolyzed, in the presence of inert solvents and, if appropriate, in the presence of acid binder.

5. Herbicidal compositions, characterized in that they contain at least one pyrimidinylthioacrylic acid derivative of the formula (I) according to Claims 1 to 4.

6. Process for combating weeds, characterized in that pyrimidinylthioacrylic acid derivative of the formula (I) according to Claims 1 to 4 are allowed to act on weeds and/or their habitat.

7. Use of pyrimidinylthioacrylic acid derivatives of the formula (I) for combating weeds.

8. Process for the preparation of herbicidal compositions, characterized in that pyrimidinylthioacrylic acid derivatives of the formula (I) according to claims 1 to 4 are mixed with extenders and/or surface active agents.

9. Pyrimidinylthioacetonitriles of the formula (VI)

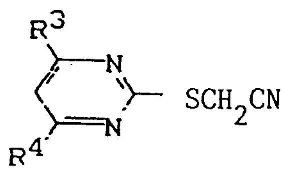

(VI)

wherein R3 and $R^4$ each independently represent $C_{1-4}$ alkyl, $C_{1-4}$ alkoxy, halogeno-$C_{1-4}$ alkyl halogeno-$C_{1-4}$ alkoxy or halogen.

**10.** Process for the preparation of pyrimidinylthioacetonitriles of the formula (VI) according to Claim 9

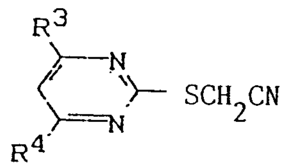

(VI)

wherein R3 and $R^4$ each independently represent $C_{1-4}$ alkyl, $C_{1-4}$ alkoxy, halogeno-$C_{1-4}$ alkyl, halogeno-$C_{1-4}$ alkoxy or halogen,
characterized in that
(f) compounds of the formula (IV)

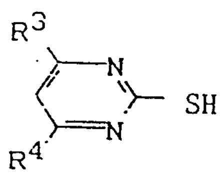

(IV)

wherein $R^3$ and $R^4$ have the same meanings as mentioned above,
are reacted with compounds of the formula (VIII)

$Y\text{-}CH_2CN$ (VIII)

wherein Y represents halogen or methylsulfonyloxy,
in the presence of an inert solvent and, if appropriate, in the presence of an acid binder.
or
(g) compounds of the formula (IX)

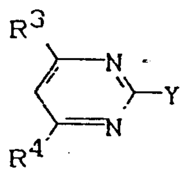

(IX)

wherein $R^3$, $R^4$ and Y have the same meanings as mentioned above,
are reacted with mercaptoacetonitrile,
if appropriate, in the presence of inert solvents and, if appropriate, in the presence of an acid binder.

**11.** Process for the preparation of compounds of the formula (IV)

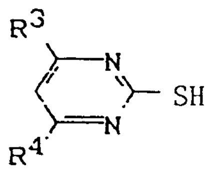

(IV)

wherein $R^3$ and $R^4$ each independently represent $C_{1-4}$ alkyl, $C_{1-4}$ alkoxy, halogeno-$C_{1-4}$ alkyl, halogeno-$C_{1-4}$ alkoxy or halogen,
characterized in that compounds of the formula ( X)

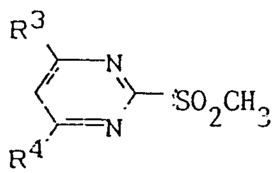

( X)

wherein $R^3$, $R^4$ and Y have the same meanings as mentioned above,
are reacted with thiolizing agents, in the presence of an inert solvent.

European Patent Office

# EUROPEAN SEARCH REPORT

Application Number

EP 93 10 7997

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 400 741 (SHELL)<br>* page 37 - page 39; claims *<br>--- | 1-8 | C07D239/60<br>A01N43/54 |
| A | EP-A-0 471 261 (BAYER)<br>* page 39; claims *<br>--- | 1-11 | |
| X | US-A-3 950 339 (A.SANTILLI ET AL.)<br>* column 1 - column 3; claim 15 *<br>--- | 9,10F | |
| X | CHEMICAL ABSTRACTS, vol. 118, 1993, Columbus, Ohio, US;<br>abstract no. 149441g,<br>E. MIKITENKO ET AL. 'CYCLAZINES AND THEIR ANALOGS. 2.'<br>page 149433 ;column 2 ;<br>* abstract *<br>& KHIM.GETEROTSIKL. SOEDIN.<br>vol. 5, 1992, KIEV<br>pages 698 - 703<br>--- | 9,10 | |
| X | D.J.BROWN 'THE PYRIMIDINES SUPPLEMENT II'<br>1986 , J.WILEY , NEW YORK<br>*CHAP.VIII PAGE 296*<br>----- | 11 | **TECHNICAL FIELDS SEARCHED (Int. Cl.5)**<br>C07D |

The present search report has been drawn up for all claims

| Place of search | Date of completion of the search | Examiner |
|---|---|---|
| THE HAGUE | 31 AUGUST 1993 | FRANCOIS J.C. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document